Europäisches Patentamt

European Patent Office

Office européen des brevets

(11) Numéro de publication: **0 321 414 B1**

(12) **FASCICULE DE BREVET EUROPEEN**

(45) Date de publication de fascicule du brevet: **09.09.92** (51) Int. Cl.⁵: **A61M 5/178**, A61M 5/315

(21) Numéro de dépôt: **88810868.5**

(22) Date de dépôt: **16.12.88**

(54) **Seringue autoprotégée.**

(30) Priorité: **16.12.87 CH 4906/87**

(43) Date de publication de la demande:
**21.06.89 Bulletin 89/25**

(45) Mention de la délivrance du brevet:
**09.09.92 Bulletin 92/37**

�ualeTitulaire: **Chanson, Albert
Chemin des Pignets 8
Ch-1028 Préverenges/VD(CH)**

㉘Etats contractants désignés:
**AT BE CH DE ES FR GB GR IT LI LU NL SE**

(56) Documents cités:
**FR-A- 2 348 708
GB-A- 2 015 883
US-A- 1 948 982**

(73) Titulaire: **Chanson, Albert
Chemin des Pignets 8
Ch-1028 Préverenges/VD(CH)**

(72) Inventeur: **Chanson, Albert
Chemin des Pignets 8
Ch-1028 Préverenges/VD(CH)**

Rank Xerox (UK) Business Services

# Description

La présente invention a pour objet une seringue autoprotégée et non réutilisable, pouvant être soit préremplie, soit remplie avant l'utilisation, à usage polyvalent, comportant un piston destiné à refouler une solution se trouvant dans le corps de la seringue sous l'action d'une tige de piston en vue de l'injection de cette solution chez un patient, un animal ou dans tout autre agent ou milieu. La partie inférieure dudit corps de seringue se prolongeant par une aiguille protégée par un capuchon, le piston étant agencé et conformé de manière à ne se déplacer sous l'action de la tige de piston que dans le sens de la poussée, c'est-à-dire du refoulement de la solution à travers l'aiguille, à ne permettre qu'une aspiration limitée à un "essai de veine", et à exclure toute autre manoeuvre, de telle sorte que la seringue devienne totalement inopérante après la première utilisation.

Les problèmes de prévention de contamination préoccupent de plus en plus non seulement les corps médicaux, mais aussi les pouvoirs publics et les organisations internationales spécialisées. De fait, la gravité actuelle de la situation devant les risques de propagation de maladies contagieuses est ressentie à l'échelle mondiale, à telle enseigne que l'Organisation Mondiale de la Santé (OMS), tout récemment, a lancé à nouveau l'idée de l'extension de l'emploi de seringues qui ne permettent véritablement qu'une seule et unique utilisation. Un autre problème, souvent soulevé, est celui des piqûres par inadvertance, celles-ci constituant une des principales causes, voire même la cause principale d'accidents dans les milieux hospitaliers. Enfin, des risques de même type doivent également être conjurés lorsque des manipulations délicates faisant intervenir des matières à haute toxicité sont effectuées, par exemple dans les industries pharmaceutiques et chimiques.

On rencontre déjà dans l'état de la technique un grand nombre de types de seringues à usage unique, c'est-à-dire ne permettant qu'une seule injection. Cependant, ces seringues, de façon générale, sont conçues de telle manière qu'un mouvement alternatif de va-et-vient reste possible. Ces seringues connues sont réutilisables au moins partiellement, moyennant, le cas échéant, une intervention simple. En réalité, les caractéristiques rencontrées sur ces seringues se trouvent en contradiction avec l'objectif à atteindre, c'est-à-dire celui d'écarter avec certitude toute réutilisation. Par ailleurs, les dispositifs connus sont complexes et donc coûteux.

Ainsi, FR-A-2 348 708 divulgue une seringue préremplie ou remplissable par le haut du corps de seringue, le piston étant hors de la seringue, non réutilisable du fait que la tige de piston devient inopérante après injection, celle-ci se détachant du piston. Il convient toutefois de remarquer que la construction telle qu'exposée dans le document en question, ne répond pas de manière optimale au but visé, puisqu'il est nécessaire d'exercer une force axiale prédéterminée pour la désolidarisation tige de piston/piston, -en-deça de laquelle le mouvement d'aspiration reste possible au-delà de celui souhaité. L'examen de la construction décrite conduit à penser que la force permettant la désolidarisation est nécessairement atteinte que par un à-coup violent. De plus, l'exercice d'une force de retrait mesurée permet, en fait, de faire remonter complètement le piston. D'autres critiques, en particulier celles relatives à l'emmanchement de la tige de piston sur le joint-piston peuvent être signalées.

De même, la construction divulguée dans GB-A-2 015 883 n'exclut nullement la réutilisation pour autant que l'utilisateur ait omis d'effectuer le premier déplacement du piston de manière complète.

Pour écarter tous ces inconvénients et pour apporter une réponse déterminante aux problèmes évoqués, la présente invention propose la réalisation d'une seringue de conception simple et rationnelle, présentant des garanties intervenant par paliers, c'est-à-dire successivement lorsque l'une d'elles fait défaut, par exemple par suite d'une manipulation intentionnelle ayant pour but de rendre la seringue réutilisable. Cet objectif est atteint au moyen d'une seringue répondant au préambule de la revendication 1, caractérisée en ce que le piston est constitué de trois éléments. L'aspiration limitée à "l'essai de veine" est obtenue par déformation de l'un des éléments du piston, à savoir un corps de piston coopérant avec les deux autres, c'est-à-dire d'une part avec un ergot central et, d'autre part, avec un joint annulaire d'étanchéité, et en ce qu'au moins un des éléments du piston est agencé de manière à se détacher de la tige de piston, respectivement de l'élément voisin lors du retrait de ladite tige, c'est-à-dire après exécution de l'injection.

Les avantages et autres caractéristiques vont ressortir de la description détaillée d'un exemple d'exécution de l'invention, basé sur le dessin annexé, dans lequel:

la fig. 1 représente une vue en coupe de la seringue,

la fig. 2 représente une coupe partielle à plus grande échelle du piston,

la fig. 3A est une vue en perspective d'un mode d'agencement de l'ergot dans l'extrémité inférieure de la tige de piston,

la fig. 3B est une vue schématique d'un autre mode d'agencement de l'ergot dans l'extrémité inférieure de la tige de piston,

la fig. 4A représente une coupe transversale de la tige de piston selon la ligne IV-IV de la fig. 1,

la fig. 4B est une coupe transversale de la tige de piston dans une autre forme d'exécution,

la fig. 5 représente une coupe longitudinale de la tige de piston agencée sur l'ajutage,

la fig. 6 représente la position et la forme du piston lors de l'"essai de veine",

la fig. 7 représente la position du piston désolidarisé de la tige de piston après utilisation,

la fig. 8 montre la désolidarisation du joint annulaire/corps de piston retenu par la tige de piston.

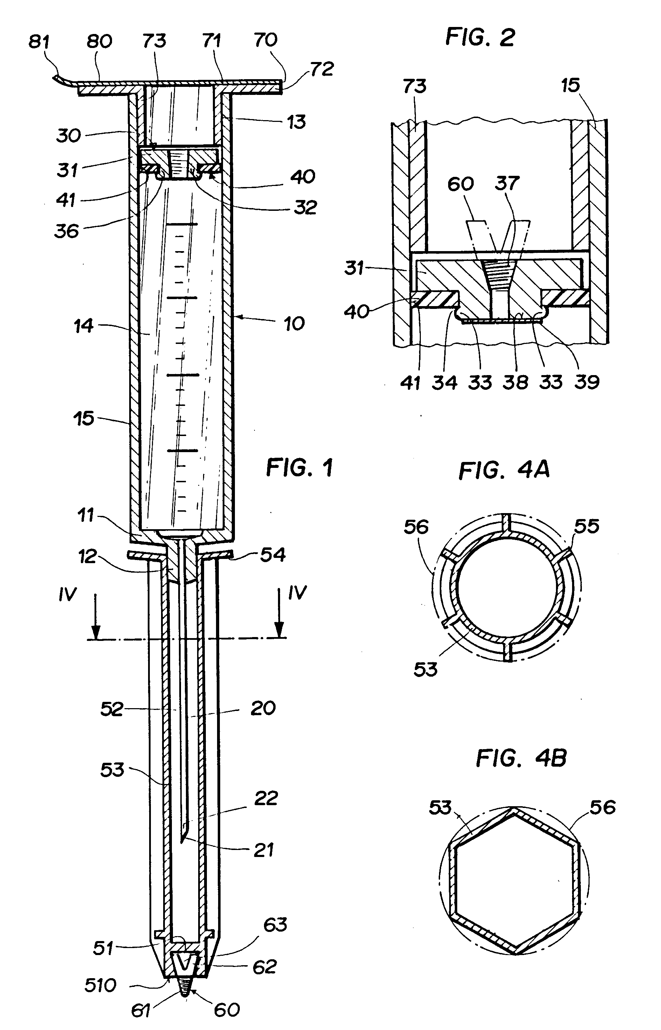

Dans la fig. 1 on aperçoit la structure générale de la seringue selon l'invention. Elle est constituée d'un corps de seringue cylindrique 10, dont l'extrémité inférieure se termine par un ajutage dans lequel est fixée, ou au bout duquel est agencée une aiguille 20, d'un piston, d'une tige de piston 50, d'une collerette 70, et d'une membrane de fermeture 80.

La seringue elle-même est présentée soit préremplie par le distributeur (producteur, fabricant, laboratoire de conditionnement des principes actifs, etc...), donc directement prête à l'emploi, soit vide, auquel cas elle sera remplie selon un procédé exposé plus loin, lorsqu'une injection est envisagée. En ce qui concerne plus particulièrement la présentation "préremplie", il importe, d'une part, que le corps de seringue, respectivement les parois du corps de seringue soient composées d'un matériau parfaitement imperméable (plastique vitrifié par exemple), et que, d'autre part, l'aiguille soit constituée de telle sorte que toutes les conditions sur le plan anti-corrosif soient respectées. Dans la partie supérieure 13 du corps de seringue, est encastrée la partie centrale 73 en forme de tube de la collerette 70 dont la face supérieure 71, non représentée en plan, peut être circulaire, ou, de préférence, en forme de losange aplati à angles arrondis, les ailes 72 situées sur la grande diagonale du losange, facilitant la "prise" de la seringue lors de sa mise en oeuvre. L'encastrement de la partie centrale 73 est obtenu selon un procédé usuel non décrit; il se prolonge sur une distance adéquate d'un cm environ, ladite partie 73 jouant dans le même temps les rôles de guide à la tige du piston 50 et de sécurité subsidiaire. L'ouverture supérieure de la seringue, quel que soit son mode de présentation, est close hermétiquement par la membrane de fermeture 80 disposée sur la face 71 de la collerette 70, afin de bien préserver la stérilisation.

La dite membrane sera aisément détachée de la collerette en soulevant et tirant simultanément l'oreille 81 qui dépasse de cette dernière. Bien entendu, tout autre moyen, tel que couvercle, capuchon, ou bouchon, s'enfichant ou s'emboîtant dans l'ouverture de la collerette, pourra être mis en oeuvre, pour réaliser cette fermeture.

Directement en-dessous du bord inférieur de la partie encastrée 73 est disposé le piston constitué de préférence, selon l'exemple décrit, de trois éléments, à savoir un corps de piston 30, un ergot central 60 et un joint annulaire d'étanchéité 40. Une coupe partielle de ce piston, à grande échelle, est représentée en fig. 2.

Le corps de piston 30, en matière plastique ou en toute autre matière, présente approximativement la forme d'un champignon, dont l'axe de révolution se superpose à l'axe du corps de seringue. Il n'est pas nécessaire que le diamètre du chapeau 31 corresponde exactement au diamètre intérieur du corps de seringue 10; au contraire, il sera de préférence choisi légèrement inférieur à ce dernier, mais supérieur au diamètre de la partie centrale 73 de la collerette 70. La hauteur du chapeau 31, quant à elle, pourra être choisie inférieure au diamètre du corps de seringue, puisque tout risque de basculement du corps de piston 30 est écarté, grâce au guidage de la tige de piston 50 mentionné plus haut.

Dans une exécution, la partie inférieure du pied 32 se termine par un léger rebord 33; la face supérieure 34 dudit rebord présentera avantageusement une forme générale arrondie ou alors tronconique dont le sommet est situé vers le haut du corps de seringue. Ce rebord, dand une variante, peut néanmoins être supprimé de telle sorte que le pied 32 se présente alors sous la forme d'un cylindre droit. Le corps de piston 30 comporte enfin un orifice ou une ouverture centrale 35. Selon la forme d'exécution préférée, celui-ci est tronconique, dans la partie 31, c.à.d. supérieure, le sommet du cône étant situé du côté de l'ajutage 12. De plus, cette partie tronconique présente un taraudage 37 à grand pas de vis à droite ou à gauche. La partie inférieure de l'ouverture, dont la hauteur correspond approximativement à celle du pied 32, est cylindrique et lisse. Elle prolonge le tronc de cône mentionné. Dans la présentation "préremplie", l'évidement central 35 sera bien sûr préalablement obturé à sa partie inférieure au moyen d'une membrane d'obturation 39 appliquée sur la face inférieure 38 du pied 32.

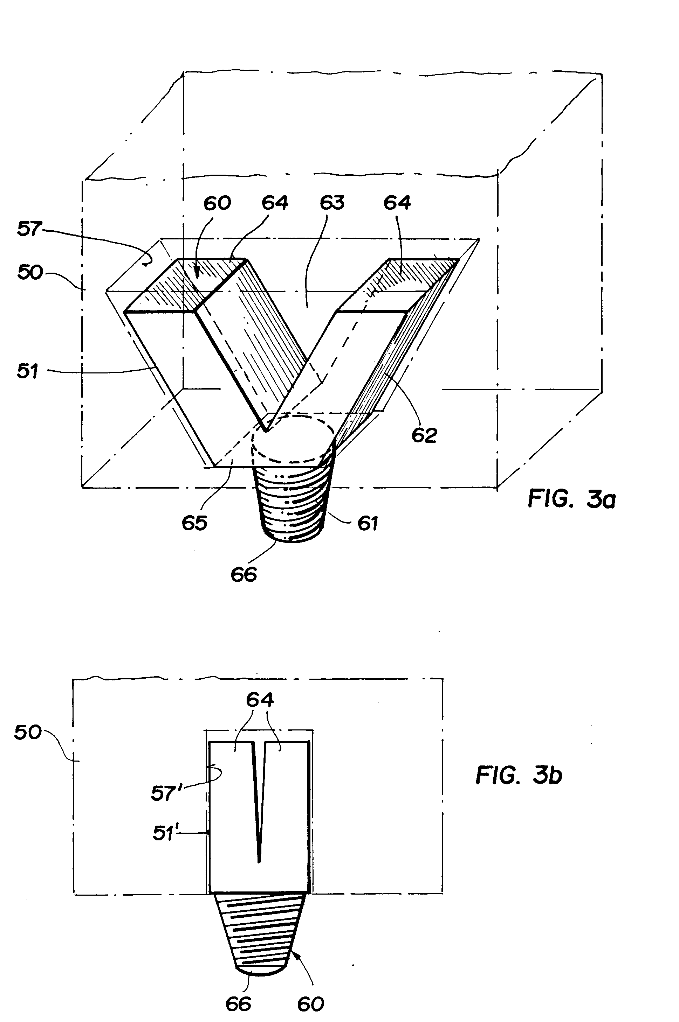

L'ouverture 35 permet, lorsque la seringue est préalablement vide (présentation "à remplir"), d'introduire la ou les solutions d'injection par le haut dans le corps de seringue, par gravitation ou tout autre moyen, lorsque l'utilisation est imminente, et est destiné à recevoir l'ergot 60 qui se présente comme une pièce massive ou partiellement massive, ayant un axe de symétrie se superposant avec celui du corps de seringue. Sur les fig. 1 et 2, cet ergot est représenté en trait mixte, en place sur l'ouverture 35. Dans tous les cas, la partie supérieure 62 de cet ergot est enfichée ou glissée auparavant dans un logement 51 de la tige de piston 50, ledit logement présentant la forme d'une queue d'aronde ainsi qu'on l'aperçoit sur la fig. 3A. Cette partie supérieure en matière souple et fendue en son milieu, forme deux lèvres 64 en forme de V d'ouverture 63. Les flancs 57 du logement 51 en queue d'aronde retiennent ces lèvres et par conséquent, l'ergot 60. La partie inférieure 61 est tronconique et présente un filetage à grand pas de vis correspondant au taraudage 37 du corps de piston. Le sommet du tronc de cône (petite base 66) se termine de préférence par une calotte sphérique. L'ergot peut être vissé dans le corps de piston jusqu'à ce que la face d'appui 65 vienne en contact avec la face supérieure dudit corps de piston.

La fig. 3B montre une autre disposition de retenue de l'ergot dans un logement 51'. Dans cette variante, le logement 51' étant parallélépipédique, les lèvres 64 de l'ergot 60 exercent une force d'appui sur les flancs 57'; l'ouverture 63 du V, dans ce cas, est quasiment fermée.

Finalement, le joint annulaire d'étanchéité 40 (fig. 1 et 2) est placé sur le pied 32, respectivement dans la gorge 36 formée par la face inférieure du chapeau 31 et la face supérieure 34 du rebord 33.

Il est clair que le bord extérieur 41 du joint annulaire 40 s'appuie uniformément contre la paroi 15 du corps de seringue 10 pour remplir la double fonction d'étanchéité et de sécurité, comme on le verra plus loin. Ledit joint assure également la fonction d'étanchéité sur la partie annulaire intérieure, c.à.d. celle en liaison avec le pied 32. Ce joint 40 sera avantageusement constitué de caoutchouc du genre néoprène ou similaire.

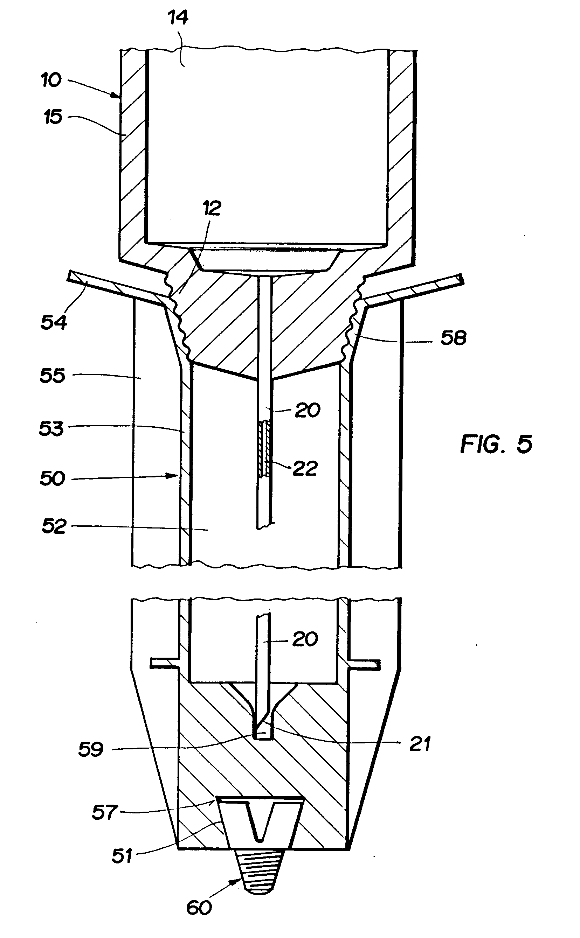

Sur l'ajutage 12 que prolonge la face inférieure du corps de seringue, est agencée la tige de piston 50, qui, à cette place, entoure l'aiguille 20. Cet agencement, ainsi que des formes préférées d'exécution de cette tige vont être décrits à l'aide des fig. 4A, 4B et 5. La tige de piston 50, se présente, dans sa forme générale, comme un tube qui, rappelons-le, est guidé par la partie 73 de la collerette lors de l'opération d'injection. Il faut souligner que, quelle que soit la section du tube (cylindrique ou polygonale), le diamètre intérieur (ou diamètre du cercle inscrit lorsqu'il s'agit d'un polygone) dudit tube n'est donc pas sensiblement inférieur au diamètre intérieur de la partie 73 de la collerette. En conséquence, le diamètre intérieur du tube sera un grand multiple - de valeur 8 par exemple - de celui de l'aiguille 20 logée dans la partie creuse 52. Les parois du tube portent la référence 53. Ainsi, cette tige tubulaire 50 remplit-elle, en plus de sa fonction de manoeuvre de piston, les rôles de capuchon-protecteur de l'aiguille 20 et celui de dispositif de sécurité, avant et après emploi de la seringue, ce qui constitue une autre caracteristique dont on saisit immédiatement l'intérêt. D'autant que l'application d'une telle tige de piston/capuchon-protecteur n'est pas obligatoirement limitée aux seringues non réutilisables, mais peut s'étendre sur tous types de seringue.

La fig. 4A est une coupe transversale de la tige de piston représentant une forme d'exécution de la section de celle-ci; la fig. 4B montre une autre variante de conformation de cette section. D'autres formes de section de la tige (tube) de piston peuvent bien entendu, être prévues. Mais quel que soit le choix de cette section, celle-ci doit être inscrite dans un cercle 56 dont la diamètre est juste inférieur au diamètre intérieur de la partie centrale 73 de la collerette 70. Dans l'exemple de la fig. 4A, le tube comporte six nervures 56, étant entendu que le nombre de nervures peut être choisi différemment; dans l'exemple de la fig. 4B, la section est hexagonale. Dans les deux cas les bords extérieurs des nervures respectivement des arêtes de l'hexagone sont inscrits dans le cercle circonscrit 56, conformément à la condition exposée plus haut. La tige, lors de l'opération d'injection, étant parfaitement guidée dès le départ, tout risque de son basculement et, dans le même temps, de celui du piston, est écarté.

La fig. 5 montre en coupe, d'une part, la partie supérieure de la tige de piston agencée sur l'ajutage 12 et, d'autre part, la partie inférieure selon un mode d'exécution préféré. La partie supérieure de la tige 50 s'ouvre en entonnoir 58, l'ajutage étant de forme correspondante, c.à.d. tronconique, le sommet du cône étant situé vers l'extrémité 21 de l'aiguille 20. Ces deux parties coniques sont pourvues d'un taraudage, respectivement d'un filetage à grand pas, de telle sorte que la tige puisse aisément, de manière sûre, fiable et rapide, être fixée sur l'ajutage, notamment après usage. L'aiguille est fixée solidairement dans l'ajutage selon un procédé connu et non décrit. La partie supérieure de la tige se termine en anneau circulaire 54 pour l'appui du pouce de la main lors de l'injection, cet anneau étant de préférence légèrement incurvé ou présentant une légère pente vers le centre. Dans d'autres variantes, les parties correspondantes (ajutage et partie supérieure de la tige) peuvent être cylindriques lisses (fig. 1) ou avec filetage, tronconiques et lisses, à section polygonale, etc. La partie inférieure, ainsi qu'il a déjà été dit lors de la description relative à l'ergot 60, comporte un logement 51 dont les flancs 57 retiennent ledit ergot par sa partie supérieure 62.

Selon la variante que représente la fig. 5, le fond de tube de la tige de piston présente une autre particularité originale, à savoir un logement 59 ménagé dans l'axe de l'aiguille 20 et dans lequel vient juste s'introduire l'extrémité 21 de ladite aiguille. L'intérêt de cette caractéristique concerne avant tout les présentations de seringues "préremplies". En effet, grâce à cette conformation, l'extrémité 21 de l'aiguille est coiffée sur tout son périmètre, une fois que la partie supérieure 58 de la tige 50 est vissée/enfichée sur l'ajutage, et que le corps de seringue contient la solution à injecter; les risques de perte inconsidérée de ladite solution sont ainsi écartés. Comme on l'aperçoit sur cette même figure, le logement 59 s'ouvre approximativement en forme de cône de guidage, le diamètre du cercle de base étant choisi suffisamment grand, de façon à faciliter au maximum l'introduction de l'extrémité de l'aiguille dans ledit logement. Bien entendu, la longueur de la partie creuse 52 de la tige 50 est dans ce cas adaptée à la longueur de l'aiguille, de telle sorte que le "coiffage" puisse être correctement assuré et que l'extrémité 21 de l'aiguille 20, ne vienne buter contre le fond du logement 59.

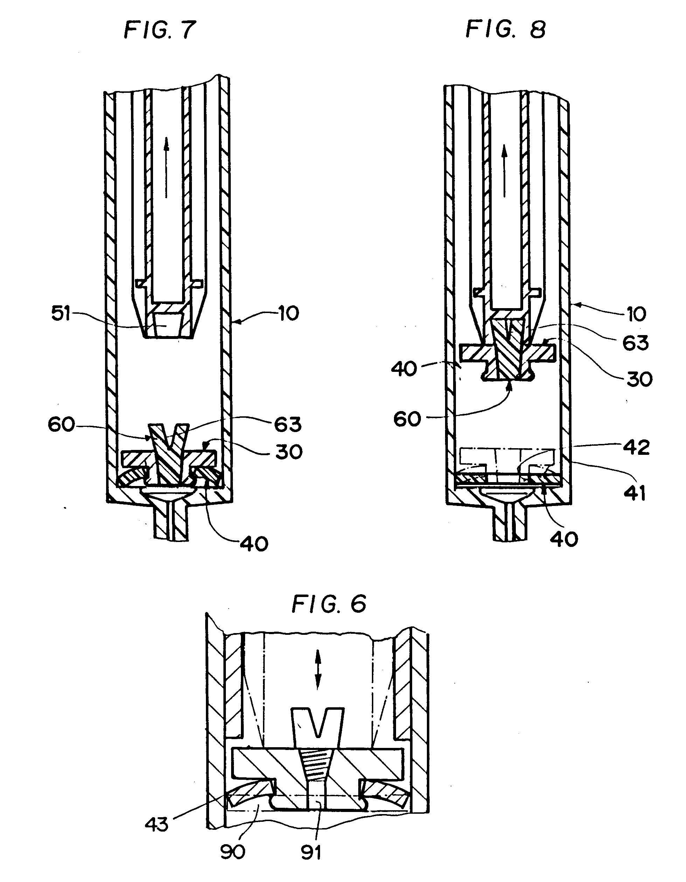

Le fonctionnement de la seringue, aisé à comprendre, va à présent être exposé à l'appui des fig. 6, 7 et 8. Rappelons que la seringue, initialement, se présente avec le piston agencé en haut du corps de seringue sous la partie centrale 73 de la collerette 70, la tige est fichée ou vissée sur l'ajutage et sert de capuchon de sécurité et de protection de stérilisation. On sait aussi que la seringue est soit préremplie (l'ouverture 35 étant alors obturée par la membrane 39), soit remplie imminemment avant l'injection, (la ou les solutions à injecter étant introduites dans le corps de seringue à travers l'ouverture 35 non obturée).

Tout d'abord, la tige de piston 50 comportant à son extrémité l'ergot 60, est libérée de l'ajutage 12, puis amenée sur le corps de piston par le haut du corps de seringue, la membrane 81 ayant préalablement été retirée. La partie inférieure 61 de l'ergot est engagée dans l'évidement central 35 du corps de piston en même temps qu'est effectué un léger mouvement de rotation, c.à.d. de vissage, de manière à bien solidariser la tige et le piston. Ce mouvement d'engagement puis de rotation est grandement facilité par la forme conique des parties 61 (1ère opération).

Il est possible dès lors de "débuller", (évacuation de l'air), en actionnant la tige 50 dans le sens du refoulement de la solution (ou des solutions) contenue(s) dans la seringue, jusqu'à ce que celle-ci commence à être évacuée de l'aiguille, ce qui implique un très court déplacement du piston vers l'ajutage (2ème opération). On est alors prêt à effecteur l'injection.

En règle générale, un "essai de veine" (3ème opération) est pratiqué avant toute injection. On sait que cet "essai de viene" a pour but de déterminer si l'aiguille, compte tenu du type de piqûre, est positionnée correctement ou non. Pour cela, une aspiration minime permet de constater la présence ou l'absence de sang dans l'aiguille. On comprend d'emblée, à l'aide de la fig. 6, comment se déroule la phase d' "essai de veine" à l'aide de la seringue selon l'invention : un mouvement minime de retrait est exercé sur la tige de piston, sans que ce mouvement ne provoque toutefois un déplacement rectiligne, à proprement dit, de l'ensemble du piston.

Grâce à sa conformation des éléments constituant le piston, on obtiendra, par ce mouvement, une première déformation du joint annulaire 40 avant même que l'on puisse observer le resserrement des lèvres 64 dans le logement 51, ainsi qu'on l'explicitera plus loin. La seule amorce de déformation du joint 40 engendre une aspiration suffisante pour le contrôle, étant précisé que l'arête extérieure supérieure 43 dudit joint lors de cette déformation se comporte comme un "pivot" qui n'est pas déplacé. (Fig. 6). De fait, le volume d'aspiration, c.à.d. celui du segment sphérique 90, ne doit que correspondre ou être juste supérieur à celui du canal 22 de l'aiguille 20. Cette condition est remplie dès que le segment 91 atteint quelques dizièmes de millimètres dans l'hypothèse d'un corps de seringue de diamètre intérieur de 12 mm, pourvu d'une aiguille de longueur 40 mm, et de diamètre intérieur (partie creuse) de 0,5 mm. L'injection (4ème opération) peut alors être pratiquée, le cas échéant après repositionnement de l'aiguille selon le résultat de l' "essai de veine".

Les fig. 7 et 8 montrent les situations postinjection. Lorsque l'opération de refoulement de la ou des solutions contenues dans le corps de seringue à travers le canal 22 de l'aiguille 20 est terminée, en d'autres termes lorsque l'injection est effectuée, l'ensemble tige de piston/piston 50, 30, 40, 60, se trouve au fond du corps de seringue. On remarque, dans ce contexte, que la forme du fond du corps de seringue épouse celle de la zone inférieure du piston, c.à.d. du joint et de la partie inférieure du pied du corps de piston, ce qui permet une évacuation optimale de la solution à injecter. On exerce alors un mouvement de retrait, c.à.d. d'aspiration sur la tige de piston, dans le sens de la flèche représentée en fig. 7 (5ème opération). A ce moment, la demi-partie inférieure 61 de l'ergot 60 reste vissée dans l'évidement 35 du corps de piston 30, tandis que la demi-partie supérieure 62 de l'ergot se détache du logement 51, ceci aussitôt après qu'il y ait eu un premier pliage du joint annulaire 40. En effet, le joint 40 s'appuie, d'une part sur la face inférieure du chapeau 31 et la face supérieure 34 du rebord 33 du corps de piston 30, et, d'autre part, latéralement sur les parois 15 du corps de seringue 10 et sur le pied 32 du corps de piston 30 (étanchéité). Les dimensions, la géométrie générale, et l'élasticité des matériaux pour la partie supérieure 62 de l'ergot 60 et du joint 40 sont déterminées par l'exigence que, lors du mouvement d'aspiration, c.à.d. de retrait de la tige de piston 50, les différents comportements ci-après apparaissent selon la séquence suivante : de manière analogue à ce qui a été exposé pour "l'essai de veine", on observe d'abord un pliage du joint 40 autour de son arête 43, lequel joint adopte une forme convexe comme on l'aperçoit sur la fig. 7. Aussitôt après, s'amorce le resserrement des lèvres 64 de l'ergot 60, le joint 40 continuant à être tenu par le rebord 33. En poursuivant le mouvement de retrait, les lèvres 64 viennent pratiquement à se rejoindre, ce qui engendre le dégagement dudit ergot de son logement 51. La première sécurité garantissant la non-réutilisation a fonctionné, la seringue devient inutilisable. Avant de jeter cette dernière, l'opérateur prendra soin de replacer ou visser la tige de piston sur l'ajutage (6ème opération). On a vu plus haut, que le diamètre de la cavité 52 (tube/tige de piston) est choisi suffisamment grand, afin que l'aiguille puisse s'introduire dans ladite cavité avec la plus grande facilité, et donc sans qu'il y ait de risque de piqûre par inadvertance lors de ce "recoiffage" de l'aiguille. La tige de piston 50 vient à nouveau remplir son rôle de capuchon protecteur d'aiguille et de sécurité post-emploi. Dans la fig. 8, on suppose que le palier de la première sécurité ait été contourné, intentionnellement, dans le but d'une tentative de réutilisation postérieure, en ayant rendue la tête de la tige de piston (50) totalement solidaire de l'ergot 60 et du corps de piston 30 par un quelconque moyen. Dans cette hypothèse, lorsque le mouvement de retrait de la tige de piston 50 est amorcé, une fois l'injection effectuée, le joint annulaire 40 accusera le premier fléchissement ainsi qu'on l'a déjà expliqué, mais la partie supérieure 62 de l'ergot restera, par la suite, fixée dans le logement 51. Lorsque l'on poursuit le retrait de la tige de piston, le joint 40, plus précisément l'arête 43 de celui-ci, est retenue par l'effet élastique contre les parois 15 du corps de seringue; le joint sera progressivement "déchaussé" du pied 32 du corps de piston. En effet, la poursuite du retrait de la tige 50 entraînera une seconde phase de fléchissement du joint 40, fléchissement qui s'accentuera toujours davantage, jusqu'à ce que le bord 42 dudit joint, finisse par glisser par-dessus le rebord 33. Le joint 40 se désolidarisera ainsi du corps de piston qui, à lui seul, est inopérant pour une utilisation ultérieure : la deuxième sécurité garantissant la non-réutilisation aura fonctionné, la seringue sera inutilisable.

Notons enfin que, dans tous les cas, il sera impossible, sauf destruction de la seringue, de retirer le corps de piston, celui-ci venant à buter contre la partie centrale 73 de la collerette 70. On comprend ainsi le rôle de sécurité subsidiaire invoqué plus haut que joue ladite collerette.

Les domaines d'application de la seringue décrite sont des plus variés, on citera à titre d'exemple les domaines médicaux, vétérinaires, de recherche, industriels. La seringue selon l'invention peut être fabriquée dans toutes les tailles et apporte une solution décisive aux problèmes exposés en introduction. Fonctionnelle et pratique, elle atteint les objectifs visés avec une réelle fiabilité, et permet en outre une fabrication aux coûts les plus bas.

## Revendications

1. Seringue auto-protégée et non réutilisable, pouvant être soit préremplie, soit remplie avant l'utilisation, à usage polyvalent, comportant un piston destiné à refouler une solution se trouvant dans le corps de la seringue sous l'action d'une tige de piston, en vue de l'injection de cette solution chez un patient, un animal ou dans tout autre agent ou milieu, la partie inférieure dudit corps de seringue se prolongeant par une aiguille protégée par un capuchon, le piston étant agencé et conformé de manière à ne se déplacer sous l'action de la tige de piston que dans le sens de la poussée, c.à d. du refoulement de la solution à travers l'aiguille, à ne permettre qu'une aspiration limitée à un "essai de veine", et à exclure toute autre manoeuvre, de telle sorte que la seringue devienne totalement inopérante après la première utilisation, caractérisée en ce que le piston est constitué de trois éléments, l'aspiration limitée à "l'essai de veine" étant obtenue par déformation de l'un (40) des éléments du piston, à savoir un corps de piston (30) coopérant avec les deux autres, c.à d. d'une part avec un ergot central (60) et, d'autre part, avec un joint annulaire d'étanchéité (40), et en ce qu'au moins un des éléments du piston (30, 40, 60) est agencé de manière à se détacher de la tige de piston (50), respectivement de l'élément voisin lors du retrait de ladite tige, c.à d. après exécution de l'injection.

2. Seringue selon la revendication 1, caractérisée en ce que le corps du piston (30) est pourvu d'une ouverture centrale (35) permettant le remplissage manuel et destinée à recevoir l'ergot (60).

3. Seringue selon la revendication 2, caractérisée en ce que l'ouverture (35) est tronconique approximativement dans sa demi-partie supérieure située vers l'extérieur du corps de seringue et présente un taraudage à grand pas de vis, et cylindrique lisse approximativement dans sa demi-partie inférieure située vers l'intérieur du corps de seringue.

4. Seringue selon la revendication 3, caractérisée en ce que le corps de piston présente approximativement la forme d'un champignon (31, 32) dont l'extrémité inférieure du pied (32) comporte un rebord (33), le joint annulaire d'étanchéité (40) étant placé sur le pied entre le chapeau (31) et ledit rebord.

5. Seringue selon la revendication 4, caractérisée en ce que l'ergot central (60) est une pièce massive dont la partie supérieure (62) coopère avec la tige de piston (50) grâce à un effet élastique, et dont la partie inférieure (61) vient s'adapter dans l'ouverture (35) du corps de piston (30).

6. Seringue selon la revendication 5, caractérisée en ce que la partie supérieure (62) de l'ergot (60) comporte deux lèvres (64) en forme de V d'ouverture (63), et en ce que la partie inférieure (61) est tronconique et filetée.

7. Seringue selon la revendication 5 ou la revendication 6, caractérisée en ce que la partie supérieure (62) de l'ergot est placée dans un logement (51) agencé dans la face frontale inférieure (510) de la tige de piston (50), les lèvres (64) dudit ergot étant retenues par les flancs (57) dudit logement.

8. Seringe selon la revendication 7, caractérisée en ce que le logement (51) présente un profil en queue d'aronde correspondant à la conformation en V de la partie supérieure (62) de l'ergot (60).

9. Seringue selon la revendication 7, caractérisée en ce que le logement (51) présente un profil droit, les flancs (57) étant parallèles à l'axe de la tige de piston (50).

## Claims

1. Self protetced and non-reusable syringe, either prefilled or filled just before utilization, polyvalent, prolongated by a needle protected by a hood, with a piston designed to move only in the injection direction, allowing only a vein test, rending the syringe inusable after the first utilization. It is characterized by its piston composed of three elements, i.e. a piston body 30 interacting with the two others, that is with the central tappet 60 on one side and with a circular rubber gasket 40 on the other, the vein test being allowed by the deformation of one of them 40, and by the fact that at least one of the elements of the piston (30,40 ,60) is designed so as to separate from the piston rod 50, respectfully from its neighbouring element during withdrawal after an injection.

2. Syringe according to patent claim 1, characterized by its central openenig 35 in the piston body 30 allowing manual filling and designed to accomodate the tappet 60.

3. Syringe according to patent claim 2, characterized by the opening 35 truncated in its upper half and fitted with a large screw tread, while being smooth cylindrical in its lower half towards the inside of the syringe body.

4. Syringe according to patent claim 3 characterized by its mushroom shaped piston body (31,32), the foot 32 of which is fitted with a shoulder 33, the circular rubber gasket 40 situated on the foot between the head 31 and the shoulder.

5. Syringe according to patent claim 4 characterized by its massive central tappet 60 whose upper part 62 is elasticly linked to the piston rod 50 and whose lower part 61 lodges into housing 35 of piston body 30.

6. Syringe according to patent claim 5 characterized by the upper side 62 of tappet 60 comporting two lips 64 forming a V shaped opening and by its lower truncated and threaded part 61.

7. Syringe according to patent claim 5 or 6 characterized by the upper part of tapet 62 being placed in a housing 51 made in the lower front side 510 of the piston rod 50, the lips 64 of the tappet being held back by the sides 57 of the above mentioned housing.

8. Syringe according to patent claim 7 characterized by its housing 51 presenting a dovetail profile corresponding to the V shape of the upper part 62 of tappet 60.

9. Syringe according to patent patent claim 7 characterized by the straight profile of housing 51, the sides of which being parallel to the axis of the piston rod 50.

## Patentansprüche

1. Eigengesicherte und nicht wiederverwendbare Injektionsspritze für vielseitige Anwendungen, die entweder vorgefüllt sein oder vor der Anwendung gefüllt werden kann und einen Kolben enthält, der dazu bestimmt ist, unter der Einwirkung eines Kolbenstössels eine Lösung, die sich im Spritzenkörper befindet, zusammenzudrücken, um diese Lösung einem Patienten oder Tier oder in irgend ein anderes Mittel oder Milieu zu injizieren, wobei der untere Teil des genannten Spritzenkörpers durch eine Nadel verlängert wird, die durch eine Kappe geschützt ist, und der Kolben so ausgeführt und ausgebildet ist, dass er sich unter der Einwirkung des Kolbenstössels nur in Drückrichtung bewegt, d.h. in Richtung des Ausströmens der Lösung durch die Nadel, dass nur eine auf eine "Venenprobe" beschränkte Ansaugung möglich ist und dass jede andere Bewegung unmöglich ist, so dass die Injektionsspritze nach der ersten Anwendung vollständig unbrauchbar wird, dadurch gekennzeichnet, dass der Kolben aus drei Elementen besteht, wobei die auf die "Venenprobe" beschränkte Ansaugung durch die Verformung eines (40) der Elemente des Kolbens erhalten wird, nämlich einem Kolbenkörper (30), der mit den beiden anderen Elementen zusammenarbeitet, d.h. einerseits mit einem zentralen Nocken (60) und andererseits mit einer ringförmigen Kolbendichtung (40), und dadurch, dass mindestens eines der Elemente des Kolbens (30, 40, 60) so eingerichtet ist, dass es sich beim Rückzug des Kolbenstössels (50), d.h. nach der Ausführung der Injektion, vom genannten Stössel beziehungsweise vom benachbarten Element löst.

2.  Injektionsspritze gemäss Anspruch 1, dadurch gekennzeichnet, dass der Kolbenkörper (30) mit einer Zentralöffnung (35) versehen ist, die das manuelle Auffüllen ermöglicht und dazu bestimmt ist, den Nocken (60) aufzunehmen.

3.  Injektionsspritze gemäss Anspruch 2, dadurch gekennzeichnet, dass die Öffnung (35) in der gegen die Aussenseite des Spritzenkörpers gerichteten oberen Hälfte ungefähr kegelstumpfförmig ist und ein Innengewinde mit einer grossen Steigung aufweist, und in der gegen die Innenseite des Spritzenkörpers gerichteten unteren Hälfte ungefähr zylindrisch und ohne Gewinde ist.

4.  Injektionsspritze gemäss Anspruch 3, dadurch gekennzeichnet, dass dieser Kolbenkörper ungefähr die Form eines Pilzes (31, 32) hat, dessen unteres Ende des Stiels (32) einen Wulst (33) aufweist, wobei die ringförmige Dichtung (40) am Stiel zwischen dem Hut (31) und dem genannten Wulst angeordnet ist.

5.  Injektionsspritze gemäss Anspruch 4, dadurch gekennzeichnet, dass dieser zentrale Nocken (60) ein massiver Teil ist, dessen oberer Teil (62) dank einem Elastizitätseffekt in den Kolbenstössel (50) eingreift, und dessen unterer Teil (61) in die Öffnung (35) des Kolbenkörpers (3) passt.

6.  Injektionsspritze gemäss Anspruch 5, dadurch gekennzeichnet, dass der obere Teil (62) des Nockens (60) zwei V-förmige Lippen (64) mit der Öffnung (63) aufweist, und dadurch, dass der untere Teil (61) kegelstumpfförmig und mit einem Aussengewinde versehen ist.

7.  Injektionsspritze gemäss Anspruch 5 oder Anspruch 6, dadurch gekennzeichnet, dass der obere Teil (62) des Nockens in einen an der unteren Frontseite (510) des Kolbenstössels (50) angeordneten Sitz (51) eingesetzt ist, wobei die Lippen (64) dieses Nockens von den Flanken (57) dieses Sitzes festgehalten werden.

8.  Injektionsspritze gemäss Anspruch 7, dadurch gekennzeichnet, dass der Sitz (51) ein schwalbenschwanzförmiges Profil hat, das der V-förmigen Ausbildung des oberen Teils (62) des Nockens (60) entspricht.

9.  Injektionsspritze gemäss Anspruch 7, dadurch gekennzeichnet, dass der Sitz (51) ein gerades Profil aufweist und dass die Flanken (57) parallel zur Achse des Kolbenstössels (50) sind.

FIG. 2

FIG. 1

FIG. 4A

FIG. 4B

FIG. 3a

FIG. 3b

FIG. 5

FIG. 7

FIG. 8

51 — 10

60 — 63
30

40

40 — 10
63
30

60

42
41

40

FIG. 6

43
90 91